# EUROPEAN PATENT APPLICATION

(11) **EP 4 631 971 A1**
(43) Date of publication of application: **15.10.2025**
(21) Application number: 23903996.9
(22) Date of filing: 13.12.2023
(51) Int. Cl.: C07K 14/34, C12N 15/77, C12P 13/14

(54) **NOVEL MANGANESE-DEPENDENT TRANSCRIPTION REGULATOR VARIANT AND METHOD FOR PRODUCING L-GLUTAMIC ACID USING SAME**

(30) Priority: 13.12.2022 KR 20220173866
(71) Applicant: CJ CheilJedang Corporation, Seoul 04560 (KR)
(72) Inventor: LEE, Heeseok, Seoul 04560 (KR); KANG, Tae-Gu, Seoul 04560 (KR); CHOI, Sun Hyoung, Seoul 04560 (KR); YOON, Byoung Hoon, Seoul 04560 (KR)
(74) Representative: Meissner Bolte Partnerschaft mbB
(86) International application number: PCT/KR2023/020540
(87) International publication number: WO 2024/128795

(57) **Abstract**

The present application pertains to a novel manganese-dependent transcription regulator variant polypeptide; a polynucleotide encoding the variant polypeptide; a microorganism containing the variant polypeptide, a polynucleotides encoding the variant polypeptide, or a vector carrying the polynucleotide; a method for producing L-glutamic acid, the method including a step of culturing the microorganism in a medium; and a use of the microorganism for the production of L-glutamic acid.

## Description

### [Technical Field]

The present disclosure relates to a novel manganese-dependent transcriptional regulator variant polypeptide; a polynucleotide encoding the variant polypeptide; a microorganism including the variant polypeptide, the polynucleotide encoding the variant polypeptide, or a vector including the polynucleotide; a method of producing L-glutamic acid, the method including the step of culturing the microorganism in a medium; and use of the microorganism for producing L-glutamic acid.

### [Background Art]

Glutamic acid, which is a representative amino acid produced by fermentation, has a unique, distinctive taste, and is one of the important amino acids widely used in the food field as well as in the medical field and other animal feed fields.

A usual method of producing glutamic acid includes production through fermentation using a microorganism of the genus *Brevibacterium* or *Corynebacterium* and Coryneform bacteria including a mutant strain thereof (Amino Acid Fermentation, Gakkai Shuppan Center: 195-215, 1986). In addition, methods of using *Escherichia coli* or microorganisms of the genera *Bacillus, Streptomyces, Penicillium, Klebsiella, Erwinia, Pantoea,* etc. are known (US Patent Publication No. 3220929 and US Patent No. 6682912).

Further, various studies to efficiently produce amino acids, for example, efforts to develop microorganisms or fermentation processes for highly efficient production of amino acids have been conducted. Specifically, target-specific approaches, such as a method of increasing expression of a gene encoding an enzyme involved in amino acid biosynthesis or a method of removing a gene unnecessary for the amino acid biosynthesis in strains of the genus *Corynebacterium* have been developed (Korean Patent Nos. 10-0924065 and 10-1208480). In addition to these methods, a method of removing a gene not involved in the production of amino acids and a method of removing a gene whose specific functions are not known with regard to the production of amino acids have also been used. However, there is still a need for research into methods of efficiently producing L-amino acids with high yields.

### [Disclosure]

### [Technical Problem]

An object of the present disclosure is to provide a microorganism capable of producing L-glutamic acid with high yields as compared to a microorganism having an existing unmodified polypeptide by culturing the microorganism including a manganese-dependent transcriptional regulator variant polypeptide, and a method of producing L-glutamic acid using the same.

### [Technical Solution]

An aspect of the present disclosure provides a manganese-dependent transcriptional regulator variant polypeptide, in which an amino acid corresponding to position 160 of SEQ ID NO: 1 is substituted with a different amino acid.

In one specific embodiment, the variant polypeptide may have substitution of aspartic acid for the amino acid corresponding to position 160 of SEQ ID NO: 1.

In another specific embodiment, the variant polypeptide may consist of an amino acid sequence of SEQ ID NO: 3.

Another aspect of the present disclosure provides a polynucleotide encoding the variant polypeptide.

Still another aspect of the present disclosure provides a microorganism including the variant polypeptide, the polynucleotide encoding the variant polypeptide, or a vector including the polynucleotide.

In one specific embodiment, the microorganism may have an increased L-glutamic acid-producing ability, as compared to a microorganism including the polypeptide of SEQ ID NO: 1 or a polynucleotide encoding the same.

With regard to the microorganism according to any one of the above specific embodiments, the microorganism may be a microorganism of the genus *Corynebacterium.*

With regard to the microorganism according to any one of the above specific embodiments, the microorganism of the genus *Corynebacterium* may be *Corynebacterium glutamicum.*

Still another aspect of the present disclosure provides a method of producing L-glutamic acid, the method including the step of culturing the microorganism in a medium.

In one specific embodiment, the method may further include the step of recovering L-glutamic acid from the cultured microorganism, a culture of the microorganism, a fermentation product of the microorganism, or the culture medium.

Still another aspect of the present disclosure provides a composition for producing L-glutamic acid, the composition including the variant polypeptide; the polynucleotide encoding the variant polypeptide; the vector including the polynucleotide; the microorganism including the variant polypeptide, the polynucleotide encoding the variant polypeptide, or the vector including the polynucleotide; or a combination of two or more thereof.

Still another aspect of the present disclosure provides use of the microorganism for producing L-glutamic acid.

### [Advantageous Effects]

When a microorganism including a manganese-dependent transcriptional regulator variant polypeptide of the present disclosure is cultured, it is possible to produce L-glutamic acid with high yields, as compared to microorganisms including the existing unmodified polypeptide.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a manganese-dependent transcriptional regulator variant polypeptide, in which an amino acid corresponding to position 160 of SEQ ID NO: 1 is substituted with a different amino acid.

As used herein, the term "manganese-dependent transcriptional regulator variant polypeptide" refers to a manganese-dependent transcriptional regulator variant polypeptide including substitution of one or more amino acids in an amino acid sequence of the manganese-dependent transcriptional regulator polypeptide; or a manganese-dependent transcriptional regulator polypeptide variant including substitution of one or more amino acids in a parent sequence that is the amino acid sequence of the manganese-dependent transcriptional regulator polypeptide.

As used herein, the term "manganese dependent transcriptional regulator (Mn-dependent transcriptional regulator, dtxR)", which is a global regulator involved in iron metabolism, refers to a factor that represses iron binding in diphtheria toxin gene expression, and may be used interchangeably with terms such as "diphtheria toxin repressor", "Tox regulatory factor", etc., and may be, but is not particularly limited to, a Mn-dependent transcriptional regulator (dtxR) encoded by the *dtxR* gene.

The gene encoding the Mn-dependent transcriptional regulator may be derived from a microorganism of the genus *Corynebacterium,* and specifically, it may be *dtxR* derived from *Corynebacterium glutamicum,* but is not limited thereto.

Specifically, examples of the Mn-dependent transcriptional regulator protein may include the amino acid sequence of SEQ ID NO: 1 or an amino acid sequence having 60% or more homology or identity thereto, but is not limited thereto, as long as they have the Mn-dependent transcriptional regulatory function. Specifically, the amino acid sequence may include SEQ ID NO: 1 or an amino acid sequence having at least 60%, 70%, 80%, 85%, 90%, 95%, 96%, 97%, 98%, or 99% or more homology or identity to SEQ ID NO: 1. The sequence of SEQ ID NO: 1 may be obtained from NCBI's GenBank or Kyoto Encyclopedia of Genes and Genomes (KEGG), which is a known database. For example, the sequence may be derived from the genus *Corynebacterium* or *Corynebacterium glutamicum,* and more specifically, it may be a polypeptide/protein including the amino acid sequence of SEQ ID NO: 1, but is not limited thereto. It is also apparent that an accessory protein having an amino acid sequence having deletion, modification, substitution, or addition of some sequence also falls within the scope of the present disclosure as long as the amino acid sequence has such homology or identity and exhibits efficacy corresponding to that of the protein.

Further, the Mn-dependent transcriptional regulator protein having the amino acid sequence of SEQ ID NO: 1 may be encoded by a polynucleotide having or including a sequence of SEQ ID NO: 2 or a nucleotide sequence having 60% or more, 70% or more, 75% or more, 80% or more, 85% or more, 90% or more, 95% or more, 96% or more, 97% or more, 98% or more, and less than 100% homology or identity to the sequence of SEQ ID NO: 2, or a polynucleotide consisting of or essentially consisting of the sequence of SEQ ID NO: 2 or a nucleotide sequence having 60% or more, 70% or more, 75% or more, 80% or more, 85% or more, 90% or more, 95% or more, 96% or more, 97% or more, 98% or more, and less than 100% homology or identity to the sequence of SEQ ID NO: 2, but is not limited thereto.

As used herein, the term "variant" refers to a polypeptide, in which one or more amino acids differ from those of the amino acid sequence before modification by conservative substitution and/or modification, but the functions or properties thereof are maintained. Such a variant may generally be identified by modifying one or more amino acids of the amino acid sequence of the polypeptide or protein and evaluating the properties of the modified polypeptide. In other words, the ability of the variant may be increased, unchanged, or decreased as compared to that of the polypeptide before being varied. Further, some variants may include variants in which one or more portions such as an N-terminal leader sequence or a transmembrane domain have been removed. Other variants may include variants in which a portion of the N- and/or C-terminus has been removed from the mature protein. The term "variant" may be used interchangeably with terms such as modification, modified polypeptide, modified protein, mutant, mutein, and divergent, and is not limited thereto as long as it is a term used with the meaning of variation.

Further, the variant may include deletions or additions of amino acids that have minimal effect on the properties and secondary structure of the polypeptide. For example, the polypeptide may be conjugated with a protein N-terminal signal (or leader) sequence that is co-translationally or post-translationally involved in the protein transfer. Further, the polypeptide may be conjugated with other sequences or linkers so as to identify, purify, or synthesize the polypeptide.

The manganese-dependent transcriptional regulator variant polypeptide of the present disclosure may be a manganese-dependent transcriptional regulator variant polypeptide in which an amino acid corresponding to position 160 of SEQ ID NO: 1 is substituted with a different amino acid, but is not limited thereto.

In one embodiment, the manganese-dependent transcriptional regulator variant polypeptide of the present disclosure may have 60% or more and less than 100% sequence homology, specifically, 80% or more and less than 100% sequence homology to the amino acid sequence of SEQ ID NO: 1, but is not limited thereto.

Specifically, the variant of the present disclosure may include substitution of the amino acid at position 160 from the N-terminus of SEQ ID NO: 1 with a different amino acid in an amino acid sequence having at least 60%, 70%, 75%, 80%, 85%, 90%, 95%, 96%, 97%, 98%, 99%, 99.5%, 99.7%, or 99.9% or more homology or identity to the amino acid sequence described by SEQ ID NO: 1. It is also apparent that a variant having an amino acid sequence having deletion, modification, substitution, conservative substitution, or addition of some sequence also falls within the scope of the present disclosure as long as the amino acid sequence has such homology or identity and exhibits efficacy corresponding to that of the variant of the present disclosure.

The "different amino acid" is not limited as long as it differs from the amino acid before substitution. Meanwhile, in the present disclosure, when expressed as 'a specific amino acid is substituted', it is obvious that the amino acid is substituted with an amino acid different from the amino acid before substitution, even though it is not separately indicated that the amino acid is substituted with a different amino acid.

The amino acid may be generally classified, based on similarity in polarity, electric charge, solubility, hydrophobicity, hydrophilicity, and/or amphipathic nature of residues.

Examples of this classification may include positively charged (basic) amino acids such as arginine, lysine, and histidine; negatively charged (acidic) amino acids such as glutamic acid and aspartic acid; amino acids with nonpolar side chains (nonpolar amino acids) such as glycine, alanine, valine, leucine, isoleucine, methionine, phenylalanine, tryptophan, and proline; amino acids with polar or hydrophilic side chains (polar amino acids) such as serine, threonine, cysteine, tyrosine, asparagine, and glutamine. For another example, amino acids may be classified into amino acids with electrically charged side chains (electrically charged amino acids) such as arginine, lysine, histidine, glutamic acid, and aspartic acid, and amino acids with uncharged side chains (uncharged amino acids; also called neutral amino acids) such as glycine, alanine, valine, leucine, isoleucine, methionine, phenylalanine, tryptophan, proline, serine, threonine, cysteine, tyrosine, asparagine, and glutamine. For still another example, phenylalanine, tryptophan, and tyrosine may be classified as aromatic amino acids. For still another example, valine, leucine, and isoleucine may be classified as branched amino acids. For still another example, 20 types of amino acids are classified according to size, starting from the amino acid group with relatively small volume, the amino acids may be classified into five groups; glycine, alanine, serine; cysteine, proline, threonine, aspartic acid, asparagine; valine, histidine, glutamic acid, glutamine; isoleucine, leucine, methionine, lysine, arginine; and phenylalanine, tryptophan, and tyrosine. However, they are not necessarily limited thereto.

For example, when it is described that "the amino acid corresponding to position 160 of SEQ ID NO: 1 is substituted with a different amino acid", it means that the amino acid is substituted with aspartic acid, valine, alanine, isoleucine, glutamic acid (glutamate), phenylalanine, arginine, cysteine, asparagine, glutamine, histidine, proline, serine, tyrosine, lysine, tryptophan, methionine, threonine, or leucine, excluding glycine, but is not limited thereto.

As used herein, although the expression "protein having an amino acid sequence described by a specific sequence number" is used, it is obvious that any protein having an amino acid sequence that includes deletion, modification, substitution, conservative substitution, or addition of some sequence may also be used in the present disclosure as long as the protein has the activity identical or equivalent to the protein consisting of the amino acid sequence of the corresponding sequence number. Examples thereof do not exclude addition of a sequence, which does not alter the function of the protein, upstream or downstream of the amino acid sequence, a mutation that may occur naturally, a silent mutation thereof, or a conservative substitution, as long as it has activity identical or corresponding to that of the variant protein, and it would be obvious that even a protein with such a sequence addition or mutation falls within the scope of the present disclosure.

The "position N" of the present disclosure may include the position N and an amino acid position corresponding to the position N. Specifically, the position N may include an amino acid position corresponding to an arbitrary amino acid residue in a mature polypeptide, disclosed in a specific amino acid sequence. The specific amino acid sequence may be the amino acid sequence of SEQ ID NO: 1.

As used herein, the term "corresponding to" refers to an amino acid residue at a position listed in a polypeptide, or an amino acid residue similar to, identical to, or homologous to a residue listed in a polypeptide. Identifying the amino acid at the corresponding position may be determining a specific amino acid in a sequence that refers to a specific sequence. As used herein, the "corresponding region" generally refers to a similar or corresponding position in a related protein or a reference protein.

For example, an arbitrary amino acid sequence is aligned with SEQ ID NO: 1, and based on this, each amino acid residue of the amino acid sequence may be numbered with reference to the numerical position of the amino acid residue corresponding to the amino acid residue of SEQ ID NO: 1. For example, a sequence alignment algorithm as described in the present disclosure may determine the position of an amino acid, or a position at which modification such as substitution, insertion, or deletion occurs through comparison with that in a query sequence (also referred to as a "reference sequence").

For such alignments, for example, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., 2000), Trends Genet. 16: 276-277), etc. may be used, but are not limited thereto, and a sequence alignment program, a pairwise sequence comparison algorithm, etc., which are known in the art, may be appropriately used.

In one specific embodiment, the variant polypeptide may have substitution of the amino acid corresponding to position 160 of SEQ ID NO: 1 with an amino acid selected from the group consisting of aspartic acid, valine, alanine, isoleucine, arginine, leucine, methionine, threonine, asparagine, glutamine, proline, serine, tryptophan, phenylalanine, histidine, cysteine, tyrosine, lysine, and glutamic acid, but is not limited thereto.

In one embodiment of the above-described embodiments, the variant polypeptide provided in the present disclosure may include substitution of the amino acid corresponding to position 160 from the N-terminus of SEQ ID NO: 1 with an amino acid selected from glutamic acid and aspartic acid, which are negatively charged (acidic) amino acids.

In one embodiment of the above-described embodiments, the variant polypeptide provided in the present disclosure may include substitution of the amino acid corresponding to position 160 from the N-terminus of SEQ ID NO: 1 with an amino acid selected from arginine, lysine, histidine, glutamic acid, and aspartic acid, which are amino acids with electrically charged side chains (electrically charged amino acid).

In one embodiment of the above-described embodiments, the variant polypeptide provided in the present disclosure may include substitution of the amino acid corresponding to position 160 from the N-terminus of SEQ ID NO: 1 with an amino acid selected from cysteine, proline, threonine, aspartic acid, and asparagine.

In one embodiment of the above-described embodiments, the manganese-dependent transcriptional regulator variant polypeptide of the present disclosure may be a polypeptide in which the amino acid corresponding to position 160 of SEQ ID NO: 1 is substituted with aspartic acid, but is not limited thereto.

For example, the variant polypeptide of the present disclosure may include an amino acid sequence having at least 60%, 70%, 75%, 80%, 85%, 90%, 95%, 96%, 97%, 98%, 99%, 99.5%, 99.7% or 99.9% or more homology or identity to SEQ ID NO: 1, provided that aspartic acid is fixed, which is the amino acid corresponding to position 160 in the amino acid sequence described by SEQ ID NO: 1. Further, it is apparent that a variant polypeptide having an amino acid sequence having deletion, modification, substitution, conservative substitution, or addition of some sequence also falls within the scope of the present disclosure as long as the amino acid sequence has efficacy corresponding to that of the variant polypeptide of the present disclosure while having such homology or identity.

Meanwhile, those skilled in the art may identify an amino acid which corresponds to the position 160 of the amino acid sequence of SEQ ID NO: 1 of the present disclosure, in an arbitrary polynucleotide sequence through sequence alignment known in the art. Unless otherwise described in the present disclosure, when "an amino acid at a specific position of a specific sequence number" is described, it is apparent that the amino acid also includes "an amino acid at the corresponding position" in an arbitrary amino acid sequence.

In another specific embodiment, the variant polypeptide may consist of an amino acid sequence of SEQ ID NO: 3.

Specifically, the variant polypeptide of the present disclosure may have or include SEQ ID NO: 3 or an amino acid sequence having at least 60%, 70%, 80%, 85%, 90%, 95%, 96%, 97%, 98%, or 99% or more homology or identity to SEQ ID NO: 3, or may consist of the amino acid sequence or may essentially consist of the amino acid sequence.

Examples thereof include those having sequence addition or deletion that does not alter the function of the variant of the present disclosure at the N-terminus or C-terminus of the amino acid sequence, and/or inside the amino acid sequence, naturally occurring mutation, silent mutation, or conservative substitution.

The "conservative substitution" means substitution of one amino acid with another amino acid having similar structural and/or chemical properties. Such an amino acid substitution may generally occur based on similarity in the polarity, charge, solubility, hydrophobicity, hydrophilicity, and/or amphipathic nature of the residues. For example, positively charged (basic) amino acids include arginine, lysine, and histidine; negatively charged (acidic) amino acids include glutamic acid and aspartic acid; aromatic amino acids include phenylalanine, tryptophan, and tyrosine; and hydrophobic amino acids include alanine, valine, isoleucine, leucine, methionine, phenylalanine, tyrosine, and tryptophan. Further, amino acids may also be classified into amino acids with electrically charged side chains and amino acids with uncharged side chains. The amino acids with electrically charged side chains include aspartic acid, glutamic acid, lysine, arginine, and histidine, and the amino acids with uncharged side chains may be classified again into nonpolar amino acids and polar amino acids. The nonpolar amino acids include glycine, alanine, valine, leucine, isoleucine, methionine, phenylalanine, tryptophan, and proline, and the polar amino acids include serine, threonine, cysteine, tyrosine, asparagine, and glutamine. Usually, conservative substitution may hardly affect or not affect the activity of produced polypeptides. Usually, conservative substitution may hardly affect or not affect the activity of proteins or polypeptides.

In one embodiment, the manganese-dependent transcriptional regulator variant polypeptide of the present disclosure may have enhanced activity of the Mn-dependent transcriptional regulator, but is not limited thereto. Further, the manganese-dependent transcriptional regulator variant polypeptide of the present disclosure may have activity to increase the L-glutamic acid-producing ability, as compared to the wild-type polypeptide of the Mn-dependent transcriptional regulator, but is not limited thereto.

Another aspect of the present disclosure provides a polynucleotide encoding the variant polypeptide.

As used herein, the term "polynucleotide" is a DNA or RNA strand having a certain length or more as a polymer of nucleotides in which nucleotide monomers are connected in a long chain by covalent bonds, and more specifically, refers to a polynucleotide fragment encoding the variant.

The polynucleotide encoding the manganese-dependent transcriptional regulator variant polypeptide of the present disclosure may include any polynucleotide sequence without limitation as long as it encodes the manganese-dependent transcriptional regulator variant polypeptide of the present disclosure. For example, the polynucleotide encoding the manganese-dependent transcriptional regulator variant polypeptide of the present disclosure may be a polynucleotide sequence encoding the amino acid sequence of the manganese-dependent transcriptional regulator variant polypeptide of the present disclosure, but is not limited thereto. In the present disclosure, the polynucleotide may be for enhancing the activity of the Mn-dependent transcriptional regulatoror for increasing the production of glutamic acid, but is not limited thereto.

For example, the polynucleotide may include a nucleotide sequence encoding the amino acid sequence described by SEQ ID NO: 3. As an example of the present disclosure, the polynucleotide of the present disclosure may have or include SEQ ID NO: 4. Further, the polynucleotide of the present disclosure may consist of or essentially consist of SEQ ID NO: 4.

The polynucleotide of the present disclosure may undergo various modifications in the coding region within the scope that does not change the amino acid sequence of the variant of the present disclosure in consideration of codon degeneracy or codons preferred in organisms that are intended to express the variant of the present disclosure. Therefore, it is obvious that, due to codon degeneracy, a polynucleotide to be translated into a polypeptide consisting of the amino acid sequence of the variant of the present disclosure or a polypeptide having homology or identity thereto may also be included. For example, the polynucleotide of the present disclosure may be SEQ ID NO: 4 or a degenerated sequence thereof.

For example, the polynucleotide of the present disclosure may include substitution of a codon encoding glycine which is an amino acid corresponding to position 479 of SEQ ID NO: 2 with a codon encoding an amino acid other than glycine, e.g., aspartic acid in a nucleotide sequence having 60%, 70%, 75%, 80%, 85%, 90%, 95%, 96%, 97%, 98%, 99%, 99.5%, 99.7% or 99.9% or more homology or identity to the sequence of SEQ ID NO: 2, but is not limited thereto. It is also apparent that a variant having a polynucleotide sequence having deletion, modification, substitution, conservative substitution, or addition of some sequence also falls within the scope of the present disclosure as long as the polynucleotide sequence has such homology or identity and encodes the amino acid sequence of the manganese-dependent transcriptional regulator variant polypeptide of the present disclosure.

For another example, the polynucleotide of the present disclosure may have or include a nucleotide sequence having 60% or more, 70% or more, 75% or more, 80% or more, 85% or more, 90% or more, 95% or more, 96% or more, 97% or more, 98% or more, and less than 100% homology or identity to SEQ ID NO: 4, or may consist of or may essentially consist of a nucleotide sequence having 60% or more, 70% or more, 75% or more, 80% or more, 85% or more, 90% or more, 95% or more, 96% or more, 97% or more, 98% or more, and less than 100% homology or identity to SEQ ID NO: 4, but is not limited thereto. Alternatively, the sequence having the above homology or identity may be a sequence in which the codon corresponding to position 160 of SEQ ID NO: 3, encoded by SEQ ID NO: 4, is fixed to the codon encoding aspartic acid.

Further, the polynucleotide of the present disclosure may include a probe that may be prepared from a known gene sequence, for example, may include any sequence without limitation as long as it is a sequence that is able to hybridize with a complementary sequence to the entirety or a part of the polynucleotide sequence of the present disclosure under stringent conditions.

The "stringent conditions" mean conditions that enable specific hybridization between polynucleotides. These conditions are specifically described in documents (see J. Sambrook et al.,Molecular Cloning, A Laboratory Manual, 2nd Edition, Cold Spring Harbor Laboratory press, Cold Spring Harbor, New York, 1989; F.M. Ausubel et al.,Current Protocols in Molecular Biology, John Wiley & Sons, Inc., New York, 9.50-9.51, 11.7-11.8). Examples thereof include a condition in which polynucleotides having higher homology or identity, i.e., polynucleotides having 60% or more, 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed once, specifically, twice to three times at a salt concentration and temperature equivalent to 60°C, 1XSSC, 0.1% SDS, specifically 60°C, 0.1XSSC, 0.1% SDS, more specifically, 68°C, 0.1XSSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also include substantially similar nucleic acid sequences as well as isolated nucleic acid fragments that are complementary to the entire sequence.

Specifically, a polynucleotide having homology or identity to the polynucleotide of the present disclosure may be detected using a hybridization condition including a hybridization step at a Tm value of 55°C and the above-described conditions. Further, the Tm value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (e.g., J. Sambrook et al., supra).

As used herein, the term "homology" or "identity" means relevance between two given amino acid sequences or nucleotide sequences and may be expressed as a percentage. The terms "homology and identity" may often be used interchangeably.

The sequence homology or identity of a conserved polynucleotide or polypeptide is determined by standard alignment algorithms, and the default gap penalty established by a program used may be used together. Substantially, homologous or identical sequences are generally capable of being hybridized with the entirety or a part of the sequence under moderately or highly stringent conditions. It is apparent that hybridization also includes hybridization of a polynucleotide with a polynucleotide including a general codon or a codon in consideration of codon degeneracy.

Whether or not any two polynucleotide or polypeptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, it may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including the GCG program package ((Devereux, J., et al, Nucleic Acids Research 12: 387(1984)), BLASTP, BLASTN, FASTA(Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, BLAST of the National Center for Biotechnology Information or ClustalW may be used to determine the homology, similarity, or identity.

The homology, similarity, or identity between polynucleotides or polypeptides may be determined by comparing sequence information using the GAP computer program as introduced by, for example, Needleman et al.(1970), J Mol Biol. 48:443, as disclosed by Smith and Waterman, Adv. Appl. Math(1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. The default parameters for the GAP program may include: (1) a binary comparison matrix (containing a value 1 for identity and a value 0 for non-identity) and the weighted comparison matrix of Gribskov et al(1986) Nucl. Acids Res. 14: 6745 as disclosed in Schwartz and Dayhoff, eds., Atlas Of Protein Sequence And Structure, National Biomedical Research Foundation, pp. 353-358(1979) (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps. Therefore, the term "homology" or "identity" used herein represents the relevance between sequences.

Still another aspect of the present disclosure provides a vector including the polynucleotide of the present disclosure. The vector may be an expression vector for expressing the polynucleotide in a microorganism, but is not limited thereto.

As used herein, the term "vector" may include a DNA construct including a nucleotide sequence of a polynucleotide encoding a polypeptide of interest which is operably linked to a suitable expression regulatory region (or expression control sequence) so that the polypeptide of interest may be expressed in a suitable host. The expression regulatory region may include a promoter capable of initiating transcription, any operator sequence for controlling the transcription, a sequence encoding a suitable mRNA ribosome binding site, and a sequence controlling termination of transcription and translation. The vector may be transformed into a suitable microorganism, and then replicated or function independently of the host genome, or may be integrated into the genome itself.

The vector used in the present disclosure is not particularly limited, but any vector known in the art may be used. Examples of commonly used vectors include natural or recombinant plasmids, cosmids, viruses, and bacteriophages. For example, pWE15, M13, MBL3, MBL4, IXII, ASHII, APII, t10, t11, Charon4A, Charon21A, etc. may be used as a phage vector or a cosmid vector, and pDZ system, pBR system, pUC system, pBluescript II system, pGEM system, pTZ system, pCL system, pET system, etc. may be used as a plasmid vector. Specifically, pDZ, pDC, pDCM2, pACYC177, pACYC184, pCL, pECCG117, pUC19, pBR322, pMW118, pCC1BAC vector, etc. may be used.

For example, a polynucleotide encoding a polypeptide of interest may be inserted into a chromosome through a vector for intracellular chromosome insertion. Insertion of the polynucleotide into the chromosome may be performed by any method known in the art, for example, homologous recombination, but is not limited thereto. The vector may further include a selection marker for the confirmation of chromosome insertion. The selection marker is for selecting the cells transformed with vectors, i.e., for confirming the insertion of a nucleic acid molecule of interest, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector including a polynucleotide encoding a target polypeptide is introduced into a microorganism or a microorganism so that the polypeptide encoded by the polynucleotide may be expressed in the microorganism. The transformed polynucleotide may be located regardless of the position, either by being inserted into the chromosome of the microorganism or located outside the chromosome as long as it may be expressed in the microorganism. Further, the polynucleotide includes DNA and/or RNA encoding a polypeptide of interest. The polynucleotide may be introduced in any form as long as it may be introduced into a microorganism and expressed. For example, the polynucleotide may be introduced into a microorganism in the form of an expression cassette, which is a gene construct containing all elements required for self-expression. The expression cassette may usually include a promoter operably linked to the polynucleotide, a transcription termination signal, a ribosome binding site, and a translation termination signal. The expression cassette may be in the form of an expression vector capable of self-replicating. Further, the polynucleotide may be introduced into a microorganism in its own form and operably linked to a sequence required for expression in the microorganism, but is not limited thereto.

Further, the term "operably linked" means that the polynucleotide sequence is functionally linked to a promoter sequence that initiates and mediates transcription of the polynucleotide encoding the desired variant of the present disclosure.

Still another aspect of the present disclosure provides a microorganism including the variant polypeptide, the polynucleotide encoding the variant polypeptide, or the vector including the polynucleotide.

**In** one specific embodiment, the microorganism of the present disclosure may be a microorganism having the L-glutamic acid-producing ability.

As used herein, the term "microorganism (or strain)" includes all of wild-type microorganisms or naturally or artificially genetically modified microorganisms, and it may be a microorganism in which a specific mechanism is weakened or strengthened due to insertion of a foreign gene or an activity enhancement or inactivation of an endogenous gene, and it may be a microorganism including genetic modification for production of a desired polypeptide, protein, or product. As used herein, the "microorganism" and "strain" have the same meaning and may be used interchangeably without limitation.

As used herein, the term "L-glutamic acid-producing microorganism" refers to a prokaryotic or eukaryotic microorganism capable of producing L-glutamic acid within a living organism, and may include both a microorganism prepared by providing the L-glutamic acid-producing ability for a parent strain without the L-glutamic acid-producing ability, or a microorganism that inherently has the L-glutamic acid-producing ability. The L-glutamic acid-producing ability may be conferred or enhanced by species improvement.

In one embodiment, the microorganism of the present disclosure may be a microorganism having naturally the manganese-dependent transcriptional regulator variant polypeptide or the L-glutamic acid-producing ability; or a microorganism in which the variant of the present disclosure or the polynucleotide encoding the same (or the vector including the polynucleotide) is introduced into and/or the L-glutamic acid-producing ability is provided for a parent strain without the manganese-dependent transcriptional regulator variant polypeptide or the L-glutamic acid-producing ability, but is not limited thereto.

In one embodiment, the microorganism of the present disclosure may include all of a microorganism including the manganese-dependent transcriptional regulator variant polypeptide sequence of the present disclosure due to mutation of the gene encoding the manganese-dependent transcriptional regulator variant polypeptide on the chromosome, and/or a microorganism including the manganese-dependent transcriptional regulator variant polypeptide of the present disclosure by introducing the vector including the polynucleotide encoding the manganese-dependent transcriptional regulator variant polypeptide of the present disclosure, but is not limited thereto.

As used herein, the term "unmodified microorganism" does not exclude strains including mutation that may occur naturally in microorganisms, and may be a wild-type strain or a natural strain itself or may be a strain before the trait is changed by genetic variation due to natural or artificial factors. For example, the unmodified microorganism may be a strain into which the manganese-dependent transcriptional regulator variant polypeptide described herein is not introduced or has not yet been introduced. The term "unmodified microorganism" may be used interchangeably with "strain before being modified", "microorganism before being modified", "unvaried strain", "unmodified strain", "unvaried microorganism", or "reference microorganism".

As used herein, the term "L-glutamic acid (L-glutamate)" refers to a kind of amino acid and is classified as a non-essential amino acid, and generally produced through fermentation of microorganisms producing glutamic acid, but is not limited thereto. L-glutamic acid is known to be the most common excitatory neurotransmitter in the central nervous system, and since L-glutamic acid has an umami taste, monosodium glutamate (MSG) has been developed therefrom and is widely used as a flavor enhancer.

The microorganism having the L-glutamic acid-producing ability of the present disclosure may be a microorganism including any one or more of the variant of the present disclosure, the polynucleotide of the present disclosure, and the vector including the polynucleotide of the present disclosure; a microorganism which is modified to express the variant of the present disclosure or the polynucleotide of the present disclosure; a microorganism (e.g., recombinant strain) expressing the variant of the present disclosure or the polynucleotide of the present disclosure; or a microorganism (e.g., recombinant strain) having the activity of the variant of the present disclosure, but is not limited thereto.
For example, the strain of the present disclosure is a cell or microorganism that is transformed with the polynucleotide of the present disclosure or the vector including the polynucleotide encoding the variant of the present disclosure to express the variant of the present disclosure, and the strain of the present disclosure may include any microorganism that is able to produce L-glutamic acid by including the variant of the present disclosure. For example, the microorganism of the present disclosure may be a recombinant strain having the increased L-glutamic acid-producing ability due to expression of the manganese-dependent transcriptional regulator variant polypeptide by introducing the polynucleotide encoding the variant of the present disclosure into a natural wild-type microorganism or a microorganism having L-glutamic acid-producing ability. The recombinant strain having the increased L-glutamic acid-producing ability may be a microorganism in which L-glutamic acid-producing ability is increased, as compared to the natural wild-type microorganism or Mn-dependent transcriptional regulator-unmodified microorganism (e.g., a microorganism expressing the wild-type Mn-dependent transcriptional regulatoror a microorganism not expressing the variant of the present disclosure), but is not limited thereto. For example, the microorganism having the increased L-glutamic acid-producing ability of the present disclosure may be a microorganism in which the L-glutamic acid-producing ability is increased, as compared to the microorganism including the polypeptide of SEQ ID NO: 1 or the polynucleotide encoding the same, but is not limited thereto. For example, the unmodified microorganism, which is the target strain being compared for whether or not the L-glutamic acid-producing ability is increased, may be a wild-type *Corynebacterium glutamicum* strain, ATCC13869 or ATCC13032 strain; or a glutamic acid-producing strain, KFCC11074 strain (KR 10-0292299 B1), but is not limited thereto.

The microorganism of the present disclosure may include any microorganism that is able to express the manganese-dependent transcriptional regulator variant polypeptide of the present disclosure by a variety of known methods in addition to introduction of the nucleotide or vector.

For example, the microorganism having the increased L-glutamic acid-producing ability may have an L-glutamic acid-producing ability of about 1% or more, specifically, about 1% or more, about 2% or more, about 3% or more, about 5% or more, about 10% or more, about 15% or more, about 20% or more, about 25% or more, about 30% or more, about 35% or more, about 40% or more, about 45% or more, about 50% or more, about 55% or more, about 60% or more, about 61% or more, about 62% or more, about 63% or more, or about 64% or more (the upper limit is not particularly limited, but may be, for example, about 200% or less, about 150% or less, about 100% or less, about 90% or less, about 80% or less, about 70% or less, or about 65% or less), as compared to that of the parent strain before modification or unmodified microorganism, but the increased amount is not limited thereto as long as the producing ability has an increased amount of a + value, as compared to the producing ability of the parent strain before modification or the unmodified microorganism. In another example, the recombinant strain having the increased L-glutamic acid-producing ability may have an L-glutamic acid-producing ability of about 1.01 times or more, about 1.02 times or more, about 1.03 times or more, about 1.05 times or more, about 1.1 times or more, about 1.15 times or more, about 1.20 times or more, about 1.25 times or more, about 1.30 times or more, about 1.35 times or more, about 1.40 times or more, about 1.45 times or more, about 1.50 times or more, about 1.55 times or more, about 1.60 times or more, about 1.61 times or more, about 1.62 times or more, about 1.63 times or more, or about 1.64 times or more (the upper limit is not particularly limited, but may be, for example, about 10 times or less, about 5 times or less, about 3 times or less, about 2 times or less, or 1.7 times or less), as compared to that of the parent strain before modification or the unmodified microorganism, but is not limited thereto. As used herein, the term "about" refers to a range which includes all of ±0.5, ±0.4, ±0.3, ±0.2, ±0.1, etc., and includes all of the values that are equivalent or similar to those following the term "about", but the range is not limited thereto.

With regard to the microorganism according to any one of the above specific embodiments, the microorganism of the present disclosure may be a microorganism of the genus *Corynebacterium* (*Corynebacteria* sp.), the genus *Escherichia* (*Escherichia* sp.), the genus *Erwinia* (Erwinia sp.), the genus *Serratia* (*Serratia* sp.), the genus *Providencia* (*Providencia* sp.), the genus *Pseudomonas* (*Pseudomonas* sp.), the genus *Leptospira* (*Leptospira* sp.), the genus *Salmonella* (*Salmonella* sp.), the genus *Brevibacteria* (*Brevibacteria* sp.), the genus *Hypomononas* (*Hypomononas* sp.), the genus *Chromobacterium* (*Chromobacterium* sp.) or the genus *Norcardia* (*Norcardia* sp.), fungi, or yeasts, specifically, a microorganism of the genus *Corynebacterium,* but is not limited thereto.

As an example of the present disclosure, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.* Specifically, the microorganism of the present disclosure may be a microorganism of the genus *Corynebacterium,* more specifically, *Corynebacterium glutamicum,* but is not limited thereto.

Meanwhile, the microorganism having the L-glutamic acid-producing ability of the present disclosure may include all of a natural wild-type microorganism itself, a microorganism with the improved L-glutamic acid-producing ability by strengthening or weakening the activity of genes related to the L-glutamic acid production mechanism, and a microorganism with the improved L-glutamic acid-producing ability by introducing or strengthening activity of a foreign gene.

Meanwhile, it was already known that the microorganism of the genus *Corynebacterium* is able to produce L-glutamic acid, but its production ability is significantly low, and the genes or mechanisms responsible for the production mechanism have not been revealed. Accordingly, the microorganism of the genus *Corynebacterium* having the L-glutamic acid-producing ability of the present disclosure may include all of a natural wild-type microorganism itself, a microorganism of the genus *Corynebacterium* with the improved L-glutamic acid-producing ability by strengthening or weakening the activity of genes related to the L-glutamic acid production mechanism, and a microorganism of the genus *Corynebacterium* with the improved L-glutamic acid-producing ability by introducing or strengthening activity of a foreign gene.

Further, the microorganism of the present disclosure may have enhanced activity of the Mn-dependent transcriptional regulator protein, as compared to that of the parent strain.

As used herein, the term, Mn-dependent transcriptional regulator "protein activity" may be used interchangeably with terms such as polypeptide activity, protein activity, etc.

As used herein, the term "enhancement" of the Mn-dependent transcriptional regulator protein activity may be used interchangeably with terms such as activation, up-regulation, overexpression, and increase, etc. Here, the activation, enhancement, up-regulation, overexpression, and increase may include both exhibiting activity that was not originally possessed and exhibiting improved activity as compared to the endogenous activity or activity before modification.

The "endogenous activity" means the activity of a specific polypeptide originally possessed by a parent strain before the trait is changed or an unmodified microorganism, when the trait is changed by genetic variation due to natural or artificial factors. This may be used interchangeably with "activity before modification". The fact that the activity of a polypeptide is "enhanced", "up-regulated", "overexpressed", or "increased" as compared to the endogenous activity means that the activity of the polypeptide is improved as compared to the activity and/or concentration (expression level) of the specific polypeptide originally possessed by a parent strain before the trait is changed or an unmodified microorganism.

The enhancement may be achieved through the introduction of a foreign polypeptide or the enhancement of activity and/or concentration (expression level) of the endogenous polypeptide. The enhancement of the activity of the polypeptide may be confirmed by an increase in the degree of activity and the expression level of the corresponding polypeptide or an increase in the amount of the product produced from the corresponding polypeptide.

For the enhancement of the activity of the polypeptide, various methods well known in the art may be applied, and the method is not limited as long as the activity of the polypeptide of interest may be enhanced as compared to that of the microorganism before being modified. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure may be:
1) increase in the intracellular copy number of the polynucleotide encoding the polypeptide;
2) replacement of a gene expression regulatory region on a chromosome encoding the polypeptide with a sequence exhibiting strong activity;
3) modification of a nucleotide sequence encoding an initiation codon or 5'-UTR region of the gene transcript encoding the polypeptide;
4) modification of the amino acid sequence of the polypeptide to enhance the activity of the polypeptide;
5) modification of the polynucleotide sequence encoding the polypeptide to enhance the activity of the polypeptide (e.g., modification of the polynucleotide sequence of the polypeptide gene to encode the polypeptide that has been modified to enhance the activity of the polypeptide);
6) introduction of a foreign polypeptide exhibiting the activity of the polypeptide or a foreign polynucleotide encoding the same;
7) codon optimization of the polynucleotide encoding the polypeptide;
8) analysis of the tertiary structure of the polypeptide to select and to modify or chemically modify the exposed site; or
9) a combination of two or more selected from 1) to 8), but is not particularly limited thereto.

More specifically, 1) the increase in the intracellular copy number of the polynucleotide encoding the polypeptide may be achieved by the introduction of, into a host cell, a vector which may replicate and function independently of the host and to which the polynucleotide encoding the corresponding polypeptide is operably linked. Alternatively, the increase may be achieved by the introduction of one copy or two or more copies of the polynucleotide encoding the corresponding polypeptide into a chromosome of a host cell. The introduction into the chromosome may be performed by introducing, into a host cell, a vector capable of inserting the polynucleotide into a chromosome of the host cell, but is not limited thereto. The vector is as described above.

2) The replacement of a gene expression regulatory region (or expression control sequence) on a chromosome encoding the polypeptide with a sequence exhibiting strong activity may be, for example, an occurrence of variation in a sequence due to deletion, insertion, nonconservative or conservative substitution, or a combination thereof, or a replacement with a sequence exhibiting stronger activity so that the activity of the expression regulatory region is further enhanced. The expression regulatory region may include, but is not particularly limited to, a promoter, an operator sequence, a sequence encoding a ribosome binding site, a sequence controlling the termination of transcription and translation, etc. For example, the replacement may be to replace the original promoter with a strong promoter, but is not limited thereto.

Examples of known strong promoters include cj1 to cj7 promoters (US 7662943 B2), a lac promoter, a trp promoter, a trc promoter, a tac promoter, a lambda phage PR promoter, a PL promoter, a tet promoter, a gapA promoter, a SPL7 promoter, a SPL13(sm3) promoter (US 10584338 B2), an O2 promoter (US 10273491 B2), a tkt promoter, an yccA promoter, etc., but is not limited thereto.

3) The modification of a nucleotide sequence encoding an initiation codon or 5'-UTR region of the gene transcript encoding the polypeptide may be, for example, substitution with a nucleotide sequence encoding another start codon having a higher polypeptide expression rate as compared to an endogenous start codon, but is not limited thereto.

4) and 5) The modification of the amino acid sequence or the polynucleotide sequence may be an occurrence of variation in the sequence due to deletion, insertion, nonconservative or conservative substitution of the amino acid sequence of the polypeptide or the polynucleotide sequence encoding the polypeptide, or a combination thereof, or a replacement with an amino acid sequence or polynucleotide sequence improved to exhibit stronger activity or an amino acid sequence or polynucleotide sequence improved to be more active so that the activity of the polypeptide is enhanced, but is not limited thereto. The replacement may be specifically performed by inserting a polynucleotide into a chromosome by homologous recombination, but is not limited thereto. The vector used here may further include a selection marker for the confirmation of chromosome insertion. The selection marker is as described above.

6) The introduction of a foreign polynucleotide exhibiting the activity of the polypeptide may be the introduction of a foreign polynucleotide into a host cell, the foreign polynucleotide encoding a polypeptide exhibiting activity identical or similar to that of the polypeptide. There is no limitation on its origin or sequence as long as the foreign polynucleotide exhibits activity identical or similar to that of the polypeptide. The method used in the introduction may be performed by appropriately selecting a known transformation method by those skilled in the art. As the introduced polynucleotide is expressed in a host cell, the polypeptide may be produced and the activity thereof may be increased.

7) The codon optimization of the polynucleotide encoding the polypeptide may be the codon optimization of an endogenous polynucleotide so as to increase transcription or translation in a host cell, or the codon optimization of a foreign polynucleotide so as to perform optimized transcription and translation in a host cell.

8) The analysis of the tertiary structure of the polypeptide to select and to modify or chemically modify the exposed site may be, for example, to determine a template protein candidate according to the degree of similarity of the sequence by comparing the sequence information of a polypeptide to be analyzed with a database storing the sequence information of known proteins, to confirm the structure based on this, and to modify or chemically modify the exposed site to be modified or chemically modified.

Such enhancement of the polypeptide activity may be an increase in the activity or concentration expression level of the corresponding polypeptide, based on the activity or concentration of the polypeptide expressed in a wild-type or a microbial strain before being modified, or an increase in the amount of a product produced from the corresponding polypeptide, but is not limited thereto.

In the microorganism of the present disclosure, partial or entire modification of the polynucleotide may be induced by (a) homologous recombination using a vector for chromosome insertion in the microorganism or genome editing using engineered nuclease (e.g., CRISPR-Cas9) and/or (b) treatment with light such as ultraviolet rays and radiation, and/or chemicals, but is not limited thereto. A method of modifying a part or the entirety of the gene may include a method of using a DNA recombination technology. For example, by introducing a nucleotide sequence or vector including a nucleotide sequence homologous to the gene of interest into the microorganism to cause homologous recombination, a part or the entirety of the gene may be deleted. The nucleotide sequence or vector to be introduced may include a dominant selection marker, but is not limited thereto.

Still another aspect of the present disclosure provides a method of producing L-glutamic acid, the method including the step of culturing the microorganism of the present disclosure in a medium.

Specifically, the method of producing L-glutamic acid of the present disclosure may include the step of culturing, in a medium, the microorganism including the variant of the present disclosure or the polynucleotide of the present disclosure or the vector of the present disclosure, but is not limited thereto.

As used herein, the term "culturing" refers to growing the microorganism of the present disclosure in appropriately adjusted environmental conditions. The culture procedure of the present disclosure may be performed according to appropriate media or culture conditions known in the art. Such culturing process may be easily adjusted and used by a person skilled in the art according to the selected strain. Specifically, the culturing may be in a batch type, a continuous type, and/or a fed-batch type, but is not limited thereto.

As used herein, the "medium" refers to a mixture containing, as main ingredients, nutrient materials required for culturing the microorganism of the present disclosure, wherein the medium supplies nutrient materials including water essential for survival and growth, growth factors, etc. Specifically, as for the media and other culture conditions used for culturing the microorganism of the present disclosure, any medium that is commonly used for culturing microorganisms may be used without particular limitation. However, the microorganism of the present disclosure may be cultured under aerobic conditions in a common medium containing appropriate carbon sources, nitrogen sources, phosphorus sources, inorganic compounds, amino acids, and/or vitamins, while controlling the temperature, pH, etc. For example, a culture medium for strains of the genus *Corynebacterium* may be found in a literature ["Manual of Methods for General Bacteriology" by the American Society for Bacteriology (Washington D.C., USA, 1981)].

In the present disclosure, the carbon source may include carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; amino acids, such as glutamic acid, methionine, lysine, etc.; and glycerol, propanediol, etc. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fishes or decomposition products thereof, defatted soybean cake or degradation products thereof, etc. may be used. These nitrogen sources may be used alone or in a combination of two or more thereof, but are not limited thereto.

The phosphate sources may include potassium phosphate monobasic, potassium phosphate dibasic, and sodium-containing salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be included. These constituent ingredients or precursors may be added to the medium in a batch or continuous manner. However, the present disclosure is not limited thereto.

The pH of the medium may be adjusted by adding compounds, such as ammonium hydroxide, potassium hydroxide, ammonia, phosphoric acid, and sulfuric acid, to the medium in an appropriate manner during culturing of the microorganism of the present disclosure. In addition, an anti-foaming agent, such as a fatty acid polyglycol ester, may be added to suppress foam formation during culturing. In addition, oxygen or oxygen-containing gas may be injected into the medium to maintain the aerobic state of the medium, or no gas may be injected or nitrogen, hydrogen or carbon dioxide gas may be injected to maintain the anaerobic or non-aerobic state of the medium, but is not limited thereto.

In the culturing of the present disclosure, the culture temperature may be maintained at 20°C to 45°C, specifically 25°C to 40°C, and the culturing may be continued until a desired amount of a product is obtained, and may be performed for about 10 hours to 160 hours, but is not limited thereto.

The L-glutamic acid produced by culturing of the present disclosure may be released into the medium or may remain in cells.

In one specific embodiment, the method of producing L-glutamic acid of the present disclosure may further include the steps of preparing the microorganism of the present disclosure, preparing a medium for culturing the strain, or a combination of these steps (regardless of the order, in any order), for example, before the culturing step.

In one specific embodiment, the method of producing L-glutamic acid of the present disclosure may further include the step of recovering the L-glutamic acid from the cultured microorganism, the culture of the microorganism, a fermentation product of the microorganism, or the culture medium. The recovering step may be further included after the culturing step.

The recovering may be collecting L-glutamic acid by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, and affinity chromatography, HPLC, and a combination of these methods may be used, and L-glutamic acid may be recovered from the medium or microorganism by using an appropriate method known in the art.

In addition, the method of producing L-glutamic acid of the present disclosure may further include a purification step. The purification may be performed by using an appropriate method known in the art. In an exemplary embodiment, when the method of producing L-glutamic acid of the present disclosure includes both the recovering step and the purification step, the recovering step and the purification step may be performed continuously or discontinuously regardless of the order, or may be performed simultaneously or integrated into one step, but is not limited thereto.

In the method of the present disclosure, the variant polypeptide, polynucleotide, L-glutamic acid, etc. are described in other aspects.

Still another aspect of the present disclosure provides a composition for producing L-glutamic acid, the composition including the variant polypeptide of the present disclosure; the polynucleotide encoding the variant polypeptide; the vector including the polynucleotide; or the microorganism including the variant polypeptide of the present disclosure, the polynucleotide encoding the variant polypeptide, or the vector including the polynucleotide; the culture of the microorganism; or a combination of two or more thereof.

The composition of the present disclosure may further include any appropriate excipient that is commonly used in the composition for producing L-glutamic acid, and examples of the excipient may include a preserving agent, a wetting agent, a dispersing agent, a suspending agent, a buffer, a stabilizing agent, an isotonic agent, etc., but are not limited thereto.

In one specific embodiment, each component present in the composition of the present disclosure may be included in a microbiologically effective amount, or in an amount that may be appropriately present in the composition for production.

In the composition of the present disclosure, the variant polypeptide, polynucleotide, L-glutamic acid, etc. are described in other aspects.

Still another aspect of the present disclosure provides use of the microorganism including the variant polypeptide of the present disclosure, the polynucleotide encoding the variant polypeptide, or the vector including the polynucleotide, for producing L-glutamic acid.

In the present disclosure, the variant polypeptide, polynucleotide, vector, microorganism, L-glutamic acid, etc. are described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1. Selection of Mutant Strains with Increased Glutamic Acid-Producing Ability through Artificial Mutation Method

### Example 1-1. Induction of Artificial Mutation through UV irradiation

In order to select a mutant strain with improved production ability of glutamic acid which is a target product of fermentation, a wild-type *Corynebacterium glutamicum* (ATCC13869) was first plated on a nutrient medium containing agar and cultured at 30°C for 16 hours. Hundreds of colonies thus obtained were irradiated with UV light at room temperature to induce random mutations in the genome of the strain.

### Example 1-2. Experiment on Fermentation titer of mutation-induced strain and Selection of the Strain

A fermentation titer test was conducted on mutant strains in which random mutations were induced in Example 1-1.

Each colony was subcultured in a nutrient medium and then cultured in a fermentation medium for 5 hours. Then, 25% tween40 was added at a concentration of 0.4% to each medium, and each colony was cultured again for 32 hours.

### <Nutrient medium>

1% glucose, 0.5% meat extract, 1% polypeptone, 0.25% sodium chloride, 0.5% yeast extract, 2% agar, 0.2% urea, pH 7.2

### <Fermentation medium>

6% glucose, 5% calcium carbonate, 2.25% ammonium sulfate, 0.1% monobasic potassium phosphate, 0.04% magnesium sulfate, 10 mg/L iron sulfate, 0.3 mg/L biotin, 0.2 mg/L thiamine hydrochloride

Each colony was cultured under the above conditions, and mutant strains producing L-glutamic acid equal to or higher than the wild-type *Corynebacterium glutamicum* (ATCC13869) were selected. Thereafter, the L-glutamic acid concentrations of the selected mutant strains were measured using HPLC. The measured L-glutamic acid concentrations are shown in Table 1 below.

**[Table 1]**

| Name of strain | L-glutamic acid (g/L) |
|---|---|
| ATCC13869 | 7.3 |
| ATCC13869-y1 | 8.9 |
| ATCC13869-y2 | 6.9 |
| ATCC13869-y3 | 7.6 |
| ATCC13869-y4 | 8.2 |
| ATCC13869-y5 | 8.4 |
| ATCC13869-y6 | 6.8 |
| ATCC13869-y7 | 6.5 |
| ATCC13869-y8 | 7.6 |
| ATCC13869-y9 | 7.2 |
| ATCC13869-y10 | 8.3 |
| ATCC13869-y11 | 9.1 |
| ATCC13869-y12 | 6.5 |
| ATCC13869-y13 | 7.4 |
| ATCC13869-y14 | 7.7 |
| ATCC13869-y15 | 7.8 |

Referring to Table 1, "ATCC13869-y1" and "ATCC13869-y11" were selected as mutant strains showing an increased glutamic acid production, as compared to the wild-type strain.

### Example 2. Identification of Mutations through Gene Sequencing

In order to identify the gene mutations of the mutant strains, the genes of the ATCC13869-y1 and ATCC13869-y11 strains selected in Example 1-2 were compared with that of the wild-type strain.
As a result, it was confirmed that the ATCC13869-y1 and ATCC13869-y11 strains had the same mutation (a nucleotide at position 479 of a polynucleotide sequence represented by SEQ ID NO: 2 was replaced by A) at a specific position in the gene *dtxR* (SEQ ID NO: 2) encoding a Mn-dependent transcriptional regulator.

Therefore, in Examples 3 and 4 below, it was intended to determine whether the mutation affects the amount of glutamic acid produced by microorganisms of the genus *Corynebacterium.*

### Example 3. Preparation of Mutation-Introduced Strains and Examination of Glutamic Acid Production

### Example 3-1. Preparation of mutation-introduced strains

It was intended to prepare mutant strains into which the mutation identified in Example 2 above was introduced. In detail, in order to introduce the mutation (the nucleotide at position 479 of the polynucleotide sequence described by SEQ ID NO: 2 was replaced by A) into the wild-type *Corynebacterium glutamicum* (ATCC13869 and ATCC13032), a vector for gene substitution was constructed to substitute glycine with aspartic acid at position 160 of the Mn-dependent transcriptional regulator represented by SEQ ID NO: 1 in the strain. A gene fragment for constructing the vector was obtained through PCR using genomic DNA of ATCC13869 as a template. Based on information about the *Corynebacterium glutamicum* (ATCC13869) gene and surrounding nucleotide sequences registered in the NIH GenBank, primers containing polynucleotides of SEQ ID NOS: 5 to 8 were prepared.

PCR was performed by denaturation at 95°C for 5 minutes, followed by 30 cycles of denaturation at 95°C for 20 seconds, annealing at 55°C for 20 seconds, and polymerization at 72°C for 30 seconds, and then polymerization at 72°C for 5 minutes. More specifically, 500 bp of a polynucleotide amplified using primers of SEQ ID NOS: 5 and 6 and 500 bp of a polynucleotide amplified using primers of SEQ ID NOS: 7 and 8 were obtained. The two gene fragments thus obtained were ligated using an infusion enzyme to a pDZ vector (Korean Patent No. 10-0924065 and International Patent Publication No. 2008-033001) which was digested with restriction enzymes BamHI and Sall, thereby preparing a gene replacement vector, which was named "pDZ-dtxR(G160D)". Information of the primer sequences used to construct the above vector is shown in Table 2 below.

**[Table 2]**

| SEQ ID NO. | Name of primer | 5'-3' sequence |
|---|---|---|
| 5 | dtxR(G160D)-AF | CGGTACCCGGGGATCCGCATAAATTGAGTTTGG |
| 6 | dtxR(G160D)-AR | ATCGATGGCACGAACGTCGGAATCAGGCTCATCTG |
| 7 | dtxR(G160D)-BF | ACGTTCGTGCCATCGATCTGCCTCTCGGTGAGAAC |
| 8 | dtxR(G160D)-BR | ATGCCTGCAGGTCGAC GGGCTTTTCCACTGATTCG |

Subsequently, the gene replacement vector was transformed into the wild-type strain by homologous recombination on the chromosome (van der Rest et al., Appl Microbiol Biotechnol 52:541-545, 1999). The strain in which the vector was inserted into the chromosome by homologous recombination was selected in a medium containing 25 mg/L of kanamycin. Thereafter, as a result of performing sequencing analysis on the *Corynebacterium glutamicum* transformant in which secondary recombination was completed, it was confirmed that the target mutation was introduced into the strain, and the strain into which the mutation was introduced was named "ATCC13869::dtxR(G160D)" and "ATCC13032::dtxR(G160D)".

### Example 3-2. Examination of glutamic acid production

The mutant strains ATCC13869::dtxR(G160D) and ATCC13032::dtxR(G160D) prepared through Example 3-1 and their respective wild-type *Corynebacterium glutamicum* (ATCC13869 and ATCC13032) were cultured in the same manner as Example 1-2.

After the culture was completed, the L-glutamic acid concentration in each medium was measured. The measured L-glutamic acid concentration is shown in Table 3 below.

**[Table 3]**

| Name of strain | L-glutamic acid (g/L) |
|---|---|
| ATCC13869 | 7.2 |
| ATCC13869::dtxR(G160D) | 9.3 |
| ATCC13032 | 3.3 |
| ATCC13032::dtxR(G160D) | 4.2 |

As shown in Table 3, it was confirmed that the concentration of L-glutamic acid produced by the *Corynebacterium glutamicum* ATCC13869::dtxR(G160D) strain into which the mutation was introduced was about 2.1 g/L (about 29%) higher than the concentration of L-glutamic acid produced by the wild-type *Corynebacterium glutamicum* ATCC13869.

It was also confirmed that the concentration of L-glutamic acid produced by the mutation-introduced *Corynebacterium glutamicum* ATCC13032::dtxR(G160D) strain was about 0.9 g/L (about 27%) higher than the concentration of L-glutamic acid produced by the wild-type *Corynebacterium glutamicum* ATCC13032.

In other words, it was confirmed that the variant of the present disclosure increases the L-glutamic acid-producing ability of microorganisms.

### Example 4. Examination of Glutamic Acid Production in Mutation-Introduced KFCC11074 Strain

### Example 4-1. Preparation of mutation-introduced strain

In order to examine whether the mutation also has the same effect in a strain with the increased glutamic acid-producing ability, in addition to the wild-type strain, it was intended to introduce the mutation into KFCC11074 strain (Korean Patent Publication No. 10-0292299), which is known as a glutamic acid-producing strain.

In detail, the pDZ-dtxR(G160D) vector prepared through Example 3-1 to replace aspartic acid with glycine at position 160 of SEQ ID NO: 1 included in the above strain was transformed into KFCC11074 strain by homologous recombination on the chromosome (van der Rest et al., Appl Microbiol Biotechnol 52:541-545, 1999). The strain in which the vector was inserted into the chromosome by homologous recombination was selected in a medium containing 25 mg/L of kanamycin. Thereafter, as a result of performing sequencing analysis on the *Corynebacterium glutamicum* transformant in which secondary recombination was completed, it was confirmed that the target mutation was introduced into the strain, and the strain into which the mutation was introduced was named "KFCC11074_dtxR(G160D)".

### Example 4-2. Examination of glutamic acid production

*Corynebacterium glutamicum* KFCC11074 into which no mutation was introduced and KFCC11074_dtxR(G160D) strain into which the mutation was introduced through Example 4-1 were cultured in the same manner as Example 1-2.

After the culture was completed, the L-glutamic acid concentration in each medium was measured. The measured L-glutamic acid concentration is shown in Table 4 below.

**[Table 4]**

| Name of strain | L-glutamic acid (g/L) |
|---|---|
| KFCC11074 | 5.4 |
| KFCC11074_ dtxR(G160D) | 8.9 |

As shown in Table 4, it was confirmed that the concentration of L-glutamic acid produced by the *Corynebacterium glutamicum* KFCC11074_dtxR(G160D) strain into which the mutation was introduced was about 3.5 g/L (about 64%) higher than the concentration of L-glutamic acid produced by *Corynebacterium glutamicum* KFCC11074 into which no mutation was introduced.

In other words, it was confirmed that the mutation of the present disclosure also increases the L-glutamic acid-producing ability of the microorganism in the strain with the increased glutamic acid-producing ability.

Taken together, the variant of the present disclosure may increase the production ability of glutamic acid, which is the target product of fermentation, by enhancing the activity of the Mn-dependent transcriptional regulator in the wild-type or glutamic acid-producing strain through the activity of the mutated gene, and accordingly, it may be useful in various industrial fields for high-yield production of glutamic acid.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A manganese-dependent transcriptional regulator variant polypeptide, wherein an amino acid corresponding to position 160 of SEQ ID NO: 1 is substituted with a different amino acid.

2. The variant polypeptide of claim 1, wherein the amino acid corresponding to position 160 of SEQ ID NO: 1 is substituted with aspartic acid.

3. The variant polypeptide of claim 1, wherein the variant polypeptide consists of an amino acid sequence of SEQ ID NO: 3.

4. A polynucleotide encoding the variant polypeptide of any one of claims 1 to 3.

5. A microorganism comprising the variant polypeptide of any one of claims 1 to 3, a polynucleotide encoding the variant polypeptide, or a vector including the polynucleotide.

6. The microorganism of claim 5, wherein the microorganism has increased L-glutamic acid-producing ability, as compared to a microorganism including the polypeptide of SEQ ID NO: 1 or a polynucleotide encoding the same.

7. The microorganism of claim 5, wherein the microorganism is a microorganism of the genus *Corynebacterium.*

8. The microorganism of claim 7, wherein the microorganism of the genus *Corynebacterium* is *Corynebacterium glutamicum.*

9. A method of producing L-glutamic acid, the method comprising the step of culturing the microorganism of claim 5 in a medium.

10. The method of claim 9, further comprising the step of recovering L-glutamic acid from the cultured microorganism, a culture of the microorganism, a fermentation product of the microorganism, or the culture medium.

11. A composition for producing L-glutamic acid, the composition comprising the variant polypeptide of any one of claims 1 to 3; a polynucleotide encoding the variant polypeptide; a vector including the polynucleotide; a microorganism including the variant polypeptide, the polynucleotide encoding the variant polypeptide, or the vector including the polynucleotide; a culture of the microorganism; or a combination of two or more thereof.

12. Use of a microorganism comprising the variant polypeptide of any one of claims 1 to 3, a polynucleotide encoding the variant polypeptide, or a vector including the polynucleotide for producing L-glutamic acid.
